# EUROPEAN PATENT SPECIFICATION

(11) **EP 2 703 378 B1**
(45) Date of publication and mention of the grant of the patent: **27.04.2016**
(21) Application number: 13161558.5
(22) Date of filing: 05.03.2009
(51) Int. Cl.: C07C 37/20, C07C 39/15, C07C 39/17, C08G 59/32

(54) **POLYPHENOLIC COMPOUNDS AND EPOXY RESINS COMPRISING CYCLOALIPHATIC MOIETIES AND PROCESS FOR THE PRODUCTION THEREOF**
POLYPHENOLISCHE VERBINDUNGEN UND EPOXIDHARZE MIT CYLCOALIPHATISCHEN TEILEN UND HERSTELLUNGSVERFAHREN DAFÜR
COMPOSÉS POLYPHÉNOLIQUES ET RÉSINES ÉPOXY COMPRENANT DES CARACTÉRISTIQUES CYCLOALIPHATIQUES ET LEUR PROCÉDÉ DE PRODUCTION

(30) Priority: 12.03.2008 US 35810 P
(43) Date of publication of application: 05.03.2014
(62) Divisional of application: 09718726.4
(73) Proprietor: Blue Cube IP LLC, Midland MI 48674 (US)
(72) Inventor: Hearn, Robert, Lake Jackson, TX Texas 77566 (US); Dettloff, Marvin, Lake Jackson, TX Texas 77566 (US); Aguirre Vargas, Fabio, Lake Jackson, TX Texas 77566 (US); Jacob, George, Lake Jackson, TX Texas 77566 (US)
(74) Representative: Bumke, Jakob Wenzel

(56) References cited:
- WO-A-89/12846
- GB-A- 942 594
- US-A- 5 146 006
- DATABASE WPI Week 198351 Thomson Scientific, London, GB; AN 1983-847918 XP002527054, & JP 58 194874 A (MITSUBISHI PETROCHEMICAL CO LTD) 12 November 1983 (1983-11-12)

## Description

### BACKGROUND OF THE INVENTION

### 1. Field of the Invention

The present invention relates generally to polyphenolic compounds and epoxy resins which comprise cycloaliphatic moieties, to processes for the production thereof, and to thermoset products which are made from these resins.

### 2. Discussion of Background Information

Electrical laminates which are used in, e.g., printed circuit boards are increasingly processed by using lead free solder. Corresponding laminates require epoxy based materials of high thermal resistance. However, epoxy based materials which display the required thermal resistance are typically highly cross-linked and display significant brittleness. Also, epoxy resins of high functionality which are required for highly cross-linked materials usually display a high viscosity in the uncured state.

### SUMMARY OF THE INVENTION

It has now unexpectedly been found that the (acid-catalyzed) condensation of cyclohexane dicarboxaldehyde (in the form of cis- and/or trans-1,3-cyclohexane dicarboxaldehyde and/or cis- and/or trans-1,4-cyclohexane dicarboxaldehyde) with phenol affords a mixture of polyphenolic compounds which upon epoxidation of phenolic hydroxy groups thereof (e.g., by reaction with epichlorohydrin) yields an epoxy resin which compared to novolac-based epoxy resins shows increased thermal resistance (as evidenced by higher glass transition and thermal decomposition temperatures) and increased toughness (as evidenced by a significantly lower rubbery modulus at temperatures above the glass transition temperature). Resins derived from other cycloaliphatic dicarboxaldehydes and/or other phenolic compounds are expected to show a similar behavior.

Accordingly, the present invention relates to a process for preparing a mixture of polyphenolic compounds. The process comprises the reaction (condensation) of a dialdehyde of a cycloalkane having from about 5 to about 24 ring carbon atoms with a phenolic compound at a ratio of phenolic hydroxy groups to aldehyde groups which affords a mixture of polyphenolic compounds which comprises at least about 20 % by weight of a polyphenolic compound of formula (I): 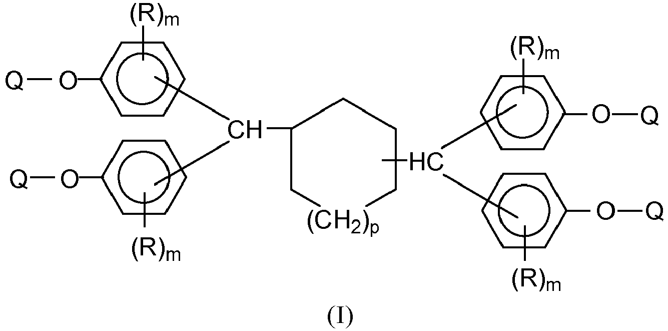 wherein:
p is 0 or an integer of from 1 to about 19;
each m independently is 0, 1, or 2;
the moieties R independently represent halogen, cyano, nitro, hydroxy, optionally substituted alkyl, optionally substituted cycloalkyl, optionally substituted alkoxy, optionally substituted alkenyl, optionally substituted alkenyloxy, optionally substituted aryl, optionally substituted aralkyl, optionally substituted aryloxy, and optionally substituted aralkyloxy; and
the moieties Q represent hydrogen;
and any non-aromatic cyclic moieties comprised in the above formula (I) may optionally carry one or more substituents and/or may optionally comprise one or more double bonds..

In one aspect of the process, the mixture of polyphenolic compounds may comprise at least about 50 % by weight of the polyphenolic compound of formula (I).

In another aspect of the process, the molar ratio of phenolic compound to cycloalkane dialdehyde may be at least about 5 : 1.

In yet another aspect of the process, the cycloalkane may have from 6 to about 19 ring carbon atoms, for example 6, 7, or 8 ring carbon atoms. Preferably, the dialdehyde comprises one or more isomers of cyclohexane dicarboxaldehyde.

In a still further aspect of the present process, the phenolic compound may comprise phenol.

The present invention also relates to a mixture of polyphenolic compounds which is obtainable by the process of the present invention as set forth above (including the various aspects thereof).

The present invention also provides a process for preparing an epoxy resin and an epoxy resin which is obtainable by this process. The process comprises partially or (substantially) completely converting phenolic hydroxy groups of the mixture of polyphenolic compounds of the present invention into glycidyl ether groups.

In one aspect thereof, the process may comprise contacting the mixture of polyphenolic compounds with epichlorohydrin.

In another aspect of the process, substantially all of the phenolic hydroxy groups may be converted into glycidyl ether groups.

The present invention also provides a (curable) mixture which comprises (i) at least one of (a) the mixture of polyphenolic compounds of the present invention and/or a prepolymerized form thereof and (b) the epoxy resin of the present invention and/or a prepolymerized form thereof and (ii) at least one of (c) a novolac resin and (d) an epoxy resin which is different from (b).

In one aspect, the third mixture may comprise an epoxy resin (d) which is obtainable by partially or substantially completely converting hydroxy groups of a novolac resin into glycidyl ether groups.

In another aspect, the third mixture may comprise a brominated epoxy resin.

In one aspect of the first to third mixtures set forth above, each of these mixtures may further comprise one or more substances which are selected from polymerization catalysts, co-curing agents, flame retardants, synergists for flame retardants, solvents, fillers, glass fibers, adhesion promoters, wetting aids, dispersing aids, surface modifiers, thermoplastic polymers, and mold release agents.

In another aspect of each of these mixtures, the corresponding mixture may be partially or completely cured.

The present invention also provides a product which comprises a first, a second and/or a third mixture of the present invention as set forth above (including the various aspects thereof) in a partially or completely cured state. For example, the product may be an electrical laminate, an IC substrate, a casting, a coating, a die attach and mold compound formulation, a composite, a potting composition, and/or an adhesive.

The present invention also provides the use of a mixture of components (a) and (b) of this invention to increase at least one of the the thermal resistance and the toughness of a resin material as described by present claim 15

The present invention also provides a polyfunctional compound of formula (I): 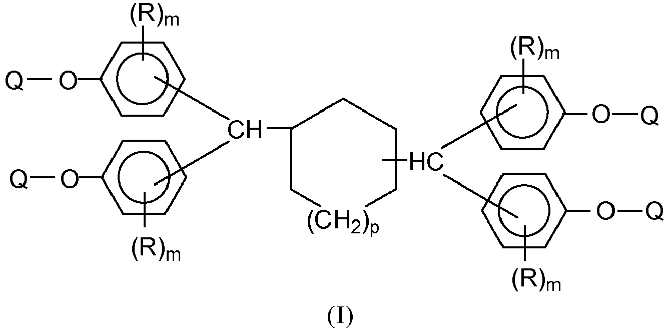 wherein:
p is 0 or an integer of from 1 to about 19;
each m independently is 0, 1, or 2;
the moieties R independently represent halogen, cyano, nitro, hydroxy, optionally substituted alkyl, optionally substituted cycloalkyl, optionally substituted alkoxy, optionally substituted alkenyl, optionally substituted alkenyloxy, optionally substituted aryl, optionally substituted aralkyl, optionally substituted aryloxy, and optionally substituted aralkyloxy; and
the moieties Q independently represent hydrogen and glycidyl;
and any non-aromatic cyclic moieties comprised in the above formula (I) may optionally carry one or more substituents and/or may optionally comprise one or more double bonds..

In one aspect, the moieties Q in the above formula (I) may be identical. For example, all moieties Q may represent hydrogen, or substantially all moieties Q may represent glycidyl groups.

In another aspect, p in the above formula may have a value of from 1 to about 14, for example, a value of 1, 2, or 3, preferably 1.

In yet another aspect of the polyfunctional compound each m in formula (I) may independently represent 0 or 1.

In a still further aspect, the polyfunctional compound may be chosen from dimethylcyclohexane tetraphenol, and dimethylcyclohexane tetraphenol tetraglycidyl ether.

Other features and advantages of the present invention will be set forth in the description of invention that follows, and will be apparent, in part, from the description or may be learned by practice of the invention. The invention will be realized and attained by the compositions, products, and methods particularly pointed out in the written description and claims hereof.

### DETAILED DESCRIPTION OF THE PRESENT INVENTION

Unless otherwise stated, a reference to a compound or component includes the compound or component by itself, as well as in combination with other compounds or components, such as mixtures of compounds.

As used herein, the singular forms "a," "an," and "the" include the plural reference unless the context clearly dictates otherwise.

Except where otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least, and not to be considered as an attempt to limit the application of the doctrine of equivalents to the scope of the claims, each numerical parameter should be construed in light of the number of significant digits and ordinary rounding conventions.

Additionally, the recitation of numerical ranges within this specification is considered to be a disclosure of all numerical values and ranges within that range. For example, if a range is from about 1 to about 50, it is deemed to include, for example, 1, 7, 34, 46.1, 23.7, or any other value or range within the range.

The particulars shown herein are by way of example and for purposes of illustrative discussion of the embodiments of the present invention only and are presented in the cause of providing what is believed to be the most useful and readily understood description of the principles and conceptual aspects of the present invention. In this regard, no attempt is made to show embodiments of the present invention in more detail than is necessary for the fundamental understanding of the present invention, the description making apparent to those skilled in the art how the several forms of the present invention may be embodied in practice.

As set forth above, the present invention provides, *inter alia*, a process for preparing a mixture of polyphenolic compounds which comprises at least about 20 % by weight of the above polyphenolic compound of formula (I) wherein Q = hydrogen. For example, the polyphenolic compound of formula (I) may account for at least about 30 %, e.g., at least about 40 %, at least about 50 %, at least about 60 %, at least about 70 %, at least about 80 %, at least about 90 %, at least about 95 %, at least about 98 %, at least about 99 %, or even about 100 % by weight of the mixture of polyphenolic compounds. The balance of the mixture of polyphenolic compounds of the present invention will usually comprise condensation products with a higher (and/or lower) degree of condensation than the polyphenolic compound of formula (I).

Preferably, the polydispersity (Mw/Mn; Mw = weight average molecular weight and Mn = number average molecular weight) of the mixture of polyphenolic compounds is not higher than about 2, e.g., not higher than about 1.8, not higher than about 1.5, or not higher than about 1.3. The average number of hydroxy groups per molecule in the mixture will usually be at least about 4, e.g., at least about 4.5 or at least about 5. Preferably, it will not be higher than about 6, e.g., not higher than about 5.5, or not higher than about 5.

The process comprises the condensation of a cycloalkane dicarboxaldehyde having from about 5 to about 24 ring carbon atoms with a phenolic compound, preferably at a ratio of phenolic compound to cycloalkane dicarboxaldeyde which affords a mixture of polyphenolic compounds with the desired polydispersity. The molar ratio of phenolic compound to cycloalkane dicarboxaldehyde employed in the reaction will usually be at least about 4 : 1 (i.e., at least about 2 phenolic hydroxy groups per one aldehyde group), e.g., at least about 4.3 : 1, or at least about 4.5 : 1. Preferably, it will be at least about 5 : 1, e.g., at least about 5.5 : 1, at least about 6 : 1, or even at least about 6.5 : 1, and may be up to about 12 : 1, up to about 15 : 1, up to about 20 : 1, or even higher. The higher the ratio of phenolic hydroxy groups to aldehyde groups the lower the extent of oligomerization that will occur, and also the lower the polydispersity and the Mw will usually be.

The cycloalkane dicarboxaldehyde which is used as a starting material in the above process may have from 5 to about 19 ring carbon atoms, e.g., up to about 12 or up to about 10 ring carbon atoms, e.g., 6, 7, 8, or 9 ring carbon atoms. For example, the cycloalkane dicarboxaldehyde may comprise one or more isomers (including regioisomers and stereoisomers) of a specific dicarboxaldehyde. By way of non-limiting example, in the case of cyclohexane dicarboxaldehyde isomers, one or more of cis-cyclohexane-1,3-dicarboxaldehyde, trans-cyclohexane-1,3-dicarboxaldehyde, cis-cyclohexane-1,4-dicarboxaldehyde and trans-cyclohexane-1,4-dicarboxaldehyde may be employed (although it is also possible to employ cis and/or trans-cyclohexane-1,2-dicarboxaldehyde). Also, a mixture of two or more dicarboxaldehydes which differ, e.g., in the number of ring carbon atoms and/or in the presence or absence, number and/or types of ring substituents (for example, a mixture of one or more cyclohexane dicarboxaldehyde isomers and one or more cyclooctane dicarboxaldehyde isomers) may be employed in the process of the present invention.

The cycloalkane moiety of the dicarboxaldehyde for use in the process of the present invention may comprise one or more (e.g., 1, 2, 3, or 4) double bonds and/or may optionally carry one or more (e.g., 1, 2, or 3) additional substituents. If more than one substituent is present, the substituents may be the same or different. Non-limiting examples of substituents which may be present on the cycloalkane ring are alkyl groups, e.g., optionally substituted alkyl groups having from 1 to about 6 carbon atoms (e.g., methyl or ethyl), optionally substituted aryl (in particular, optionally substituted phenyl), and halogen atoms such as, e.g., F, Cl, and Br. The alkyl and aryl groups may be substituted with, e.g., one or more halogen atoms such as, e.g., F, Cl, and Br.

The phenolic compound for use in the process of the present invention may be (unsubstituted) phenol. Moreover, the aromatic ring of phenol may comprise one or more (e.g., 1, 2, 3, or 4) substituents, for example one or two substituents. If two or more substituents are present, they may be the same or different. Non-limiting examples of substituents which may be present on the phenol ring are halogen (e.g., F, Cl, and Br, preferably Cl or Br), cyano, nitro, hydroxy, unsubstituted or substituted alkyl preferably having from 1 to about 6 carbon atoms, unsubstituted or substituted cycloalkyl preferably having from about 5 to about 8 carbon atoms, unsubstituted or substituted alkoxy preferably having from 1 to about 6 carbon atoms, unsubstituted or substituted alkenyl preferably having from 3 to about 6 carbon atoms, unsubstituted or substituted alkenyloxy preferably having from 3 to about 6 carbon atoms, unsubstituted or substituted aryl preferably having from 6 to about 10 carbon atoms, unsubstituted or substituted aralkyl preferably having from 7 to about 12 carbon atoms, unsubstituted or substituted aryloxy preferably having from 6 to about 10 carbon atoms, and unsubstituted or substituted aralkoxy preferably having from 7 to about 12 carbon atoms.

It is to be appreciated that whenever the terms "alkyl" and "alkenyl" are used in the present specification and the appended claims, these terms also include the corresponding cylcoaliphatic groups such as, e.g., cyclopentyl, cyclohexyl, cyclopentenyl, and cyclohexenyl. Also, where two alkyl and/or alkenyl groups are attached to two carbon atoms of an aliphatic or aromatic ring, they may be combined to form an alkylene or alkenylene group which together with the carbon atoms to which this group is attached results in a preferably 5- or 6-membered ring structure. In the case of non-adjacent carbon atoms, this ring structure may give rise to a bicyclic compound.

The above alkyl groups and alkoxy groups will often comprise from 1 to about 4 carbon atoms and in particular, 1 or 2 carbon atoms. Non-limiting specific examples of these groups include methyl, ethyl, propyl, isopropyl, n-butyl, isobutyl, sec-butyl, and tert-butyl, and methoxy, ethoxy, propoxy, isopropoxy, n-butoxy, isobutoxy, sec-butoxy, and tert-butoxy. The alkyl and alkoxy groups may be substituted with one or more (e.g., 1, 2, or 3) substituents. If more than one substituent is present, the substituents may be the same or different and are preferably identical. Non-limiting examples of these substituents include halogen atoms such as, e.g., F, Cl, and Br. Non-limiting examples of substituted alkyl and alkoxy groups include CF₃, CF₃CH₂, CC1₃, CCl₃CH₂, CHCl₂, CH₂Cl, CH₂Br, CCl₃O, CHCl₂O, CH₂ClO, and CH₂BrO.

The above alkenyl and alkenyloxy groups will often comprise 3 or 4 carbon atoms and in particular, 3 carbon atoms. Non-limiting specific examples of these groups are allyl, methallyl and 1-propenyl. The alkenyl and alkenyloxy groups may be substituted with one or more (e.g., 1, 2, or 3) substituents. If more than one substituent is present, the substituents may be the same or different and are preferably identical. Non-limiting examples of these substituents include halogen atoms such as, e.g., F, Cl, and Br.

The above aryl and aryloxy groups will often be phenyl and phenoxy groups. The aryl and aryloxy groups may be substituted with one or more (e.g., 1, 2, 3, 4, or 5) substituents. If more than one substituent is present, the substituents may be the same or different. Non-limiting examples of these substituents include nitro, cyano, halogen such as, e.g., F, Cl, and Br, optionally halogen-substituted alkyl having from 1 to about 6 carbon atoms, e.g., from 1 to about 4 carbon atoms (for example, methyl or ethyl) and optionally halogen-substituted alkoxy having from 1 to about 6 carbon atoms, e.g., from 1 to about 4 carbon atoms (for example, methoxy or ethoxy). Non-limiting specific examples of substituted aryl and aryloxy groups include, tolyl, xylyl, ethylphenyl, chlorophenyl, bromophenyl, tolyloxy, xylyloxy, ethylphenoxy, chlorophenoxy, and bromophenoxy.

The above aralkyl and aralkoxy groups will often be benzyl, phenethyl, benzyloxy, or phenethoxy groups. These groups may be substituted (preferably on the aryl ring, if at all) with one or more (e.g., 1, 2, 3, 4 or 5) substituents. If more than one substituent is present, the substituents may be the same or different. Non-limiting examples of these substituents include nitro, cyano, halogen such as, e.g., F, Cl, and Br, optionally halogen-substituted alkyl having from 1 to about 6 carbon atoms, e.g., from 1 to about 4 carbon atoms (for example, methyl or ethyl), and optionally halogen-substituted alkoxy having from 1 to about 6 carbon atoms, e.g., from 1 to about 4 carbon atoms (for example, methoxy, or ethoxy).

Of course, as in the case of the dicarboxaldehyde, two or more different phenolic compounds may be employed in the process of the present invention (e.g., phenol and a substituted phenol or two differently substituted phenol compounds), although this is usually not preferred.

The cycloaliphatic dicarboxaldehydes which are starting materials for the process for preparing the mixture of polyphenolic compounds of the present invention may be prepared by methods which are well known to those of skill in the art. By way of non-limiting example, cyclohexane (1,3 and/or 1,4)- dicarboxaldehyde can be produced, e.g., by hydroformylation of a cyclohexene carboxaldehyde, which in turn can be prepared by a Diels-Alder reaction of a conjugated diene such as, e.g., butadiene, piperylene, isoprene and chloroprene with an optionally substituted alpha,beta-unsaturated aldehyde such as, e.g., acrolein, methacrolein, crotonaldehyde or cinnamaldehyde as the dienophile. In this regard, U.S. Patent No. 6,252,121 and Japanese patent application JP 2002-212109, the entire disclosures whereof are incorporated by reference herein, may, for example, be referred to. Other relevant prior art is disclosed in US-A-5146006 and WO-A-8912846. These (in no way limiting) reactions may be schematically represented as follows:

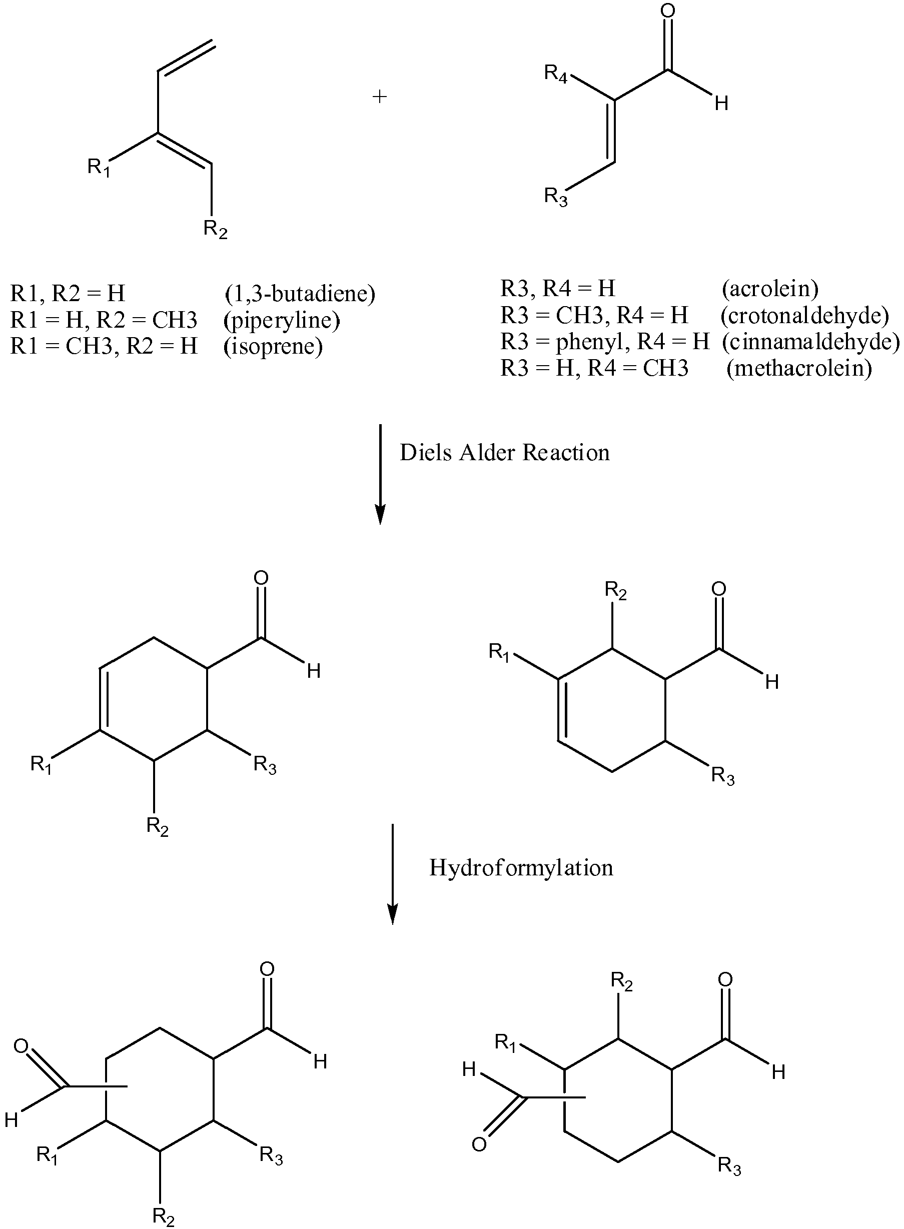

By using cyclic dienes such as, e.g., cyclopentadiene, cyclohexadiene or furan as conjugated diene in the Diels-Alder reaction, bicyclic unsaturated aldehydes may be obtained, as illustrated in the following scheme:

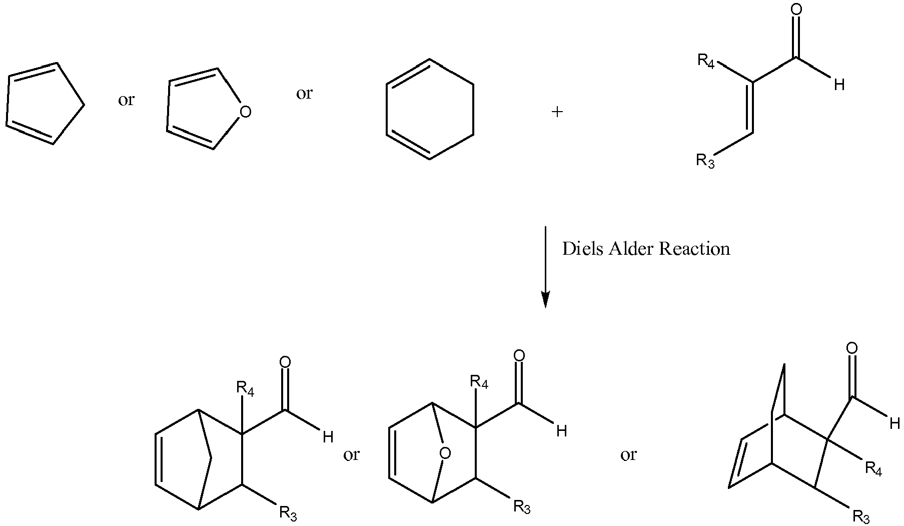

Cycloaliphatic dicarboxaldehydes may also be prepared by hydroformylation of cyclic diolefins such as, e.g., cyclooctadiene, as described in, for example U.S. Patent No. 5,138,101 and DE 198 14 913, or by ozonolysis of bicyclic olefins such as norbornene to produce cyclopentane dicarboxaldehyde (see, e.g., Perry, J. Org. Chem., 42, 829-833, 1959). The entire disclosures of these three documents are incorporated by reference herein.

By way of non-limiting example, the condensation of one or more (optionally substituted) cyclohexane dicarboxaldehydes with an (optionally substituted) phenol affords a mixture of polyphenolic compounds which comprises one or more isomers of (optionally substituted) cyclohexane dicarboxaldehyde tetraphenol along with compounds with a higher (and lower) degree of condensation.

In the above formula (I), p is 0 or an integer of from 1 to about 19, e.g., up to about 14, up to about 12 or up to about 8 such as, e.g., 1, 2, 3, 4, 5, 6, and 7, with 1, 2, or 3 being preferred and 1 being particularly preferred.

The central cycloaliphatic moiety in the above formula (I) may comprise one or more (e.g., 1, 2, 3, or 4) double bonds and/or may carry one or more (e.g., 1, 2 or 3) substituents (although the cycloaliphatic moiety will usually not comprise any double bonds). If more than one substituent is present, the substituents may be the same or different. Non-limiting examples of substituents which may be present on the central cycloaliphatic moiety have been set forth above.

The value of each m in the above formula (I) independently is 0, 1, or 2. Preferably, the values of m are identical. Also preferably, m equals 0 or 1.

The moieties R in the above formula (I) independently represent halogen (e.g., F, Cl, and Br, preferably Cl or Br), cyano (-CN), nitro, hydroxy, unsubstituted or substituted alkyl preferably having from 1 to about 6 carbon atoms, unsubstituted or substituted alkoxy preferably having from 1 to about 6 carbon atoms, unsubstituted or substituted alkenyl preferably having from 3 to about 6 carbon atoms, unsubstituted or substituted alkenyloxy preferably having from 3 to about 6 carbon atoms, unsubstituted or substituted aryl preferably having from 6 to about 10 carbon atoms, unsubstituted or substituted aralkyl preferably having from 7 to about 12 carbon atoms, unsubstituted or substituted aryloxy preferably having from 6 to about 10 carbon atoms, and unsubstituted or substituted aralkoxy preferably having from 7 to about 12 carbon atoms.

Regarding exemplary and preferred meanings of the moieties R the comments set forth above with respect to the substituents on the substituted phenol starting material of the process of the present invention apply in their entirety and may be referred to.

For the preparation of the mixture of polyphenolic compounds of the present invention reaction conditions which are conventional for the preparation of novolac resins may, for example, be used (with the exception of the ratio of the number of phenolic hydroxy groups to the number of aldehyde groups which is typically much higher in the process of the present invention than in the preparation of a novolac resin). By way of non-limiting example, reaction temperatures of from about 20°C to about 80°C may be used. As acidic catalysts for catalyzing the reaction between the dicarboxaldehyde(s) and the phenolic compound(s) inorganic and organic acids may be used such as, e.g., those which are conventionally used in the preparation of (formaldehyde-based) novolac resins. A particularly preferred acidic catalyst for use in the process of the present invention is p-toluene sulfonic acid. If at least one of the reactants is a liquid at the reaction temperature the use of a solvent may be dispensed with, although solvents may, of course, be used.

Regarding suitable reaction conditions for the production of polyphenolic compounds by reacting phenolic compounds with aldehydes which are different from formaldehyde U.S. Patent No. 5,012,016 and Kirk-Othmer, Encyclopedia of Chemical Technology, 1996, John Wiley & Sons, chapter "Phenolic Resins" (author: Peter Kopf), volume 18, pp. 603-644, the entire disclosures whereof are incorporated by reference herein, may, for example, by referred to.

The present process is very versatile as far as the mixture of polyphenolic compounds obtainable thereby is concerned. For example, a very low polydispersity product mixture with a high average functionality can be produced by this process. By way of non-limiting example, when cyclohexane dicarboxaldehyde and phenol are employed as starting materials in the process of the present invention, products having a weight average molecular weight (Mw) of about 930 and a number average molecular weight (Mn) of about 730 and/or an average of about 6 hydroxy groups per molecule can be produced by using a relatively high ratio of phenolic hydroxy groups to aldehyde functionalities to keep the degree of oligomerization low. The excess phenolic starting material may then be removed, for example, by distillation.

The conversion of the hydroxy groups of the mixture of polyphenolic compounds of the present invention into glycidyl ether groups (i.e., groups of formula-O-CH₂-CH(O)CH₂) to produce an epoxy resin is possible by using, for example, conventional processes. Usually at least about 60%, e.g., at least about 70%, at least about 80%, at least about 90%, at least about 95%, at least about 98%, at least about 99% or even substantially all (about 100%) of the phenolic hydroxy groups of the mixture of polyphenolic compounds will be converted into glycidyl ether groups.

By way of non-limiting example, for preparing an epoxy resin the mixture of polyphenolic compounds prepared by the process of the present invention may be reacted with epichlorohydrin in the presence of a base and optionally in the presence of a solvent. The epichlorohydrin will usually be employed in an at least about stoichiometric amount with respect to the hydroxy groups which are present in the mixture of polyphenolic compounds. In particular, the ratio of the number of epoxy groups of the epichlorohydrin to the number of the hydroxy groups which are present in the mixture of polyphenolic compounds will often be at least about 2 : 1, e.g., at least about 2.5 : 1, at least about 3 : 1, at least about 4 : 1, or at least about 5 : 1, but will usually be not higher than about 30 : 1, e.g., not higher than about 20 : 1, not higher than about 15 : 1, or not higher than about 12 : 1.

Non-limiting examples of bases for use in the above reaction are inorganic bases such as alkali and alkaline earth hydroxides. NaOH and KOH are examples of preferred base materials. The equivalent ratio of base to the hydroxy groups which are present in the mixture of polyphenolic compounds will usually be at least about 0.9 : 1, e.g., at least about 0.95 : 1, or at least about 0.98 : 1, but will usually be not higher than about 1.2 : 1, e.g., not higher than about 1.1 : 1, or not higher than about 1.05 : 1.

Usually reaction temperatures of from about 20°C to about 85°C will be employed, e.g., reaction temperatures of from about 40°C to about 80°C or from about 50°C to about 70°C.

Reaction times can vary substantially, for example, as a function of the reactants being employed, the reaction temperature, solvent(s) used, the scale of the reaction, and the like, but are often in the range of from about 2 hours to about 6 hours, e.g., from about 3 hours to about 5 hours.

The epoxidation reaction can be carried out with or without solvent (in the latter case epichlorohydrin may serve also as the reaction medium). Non-limiting examples of suitable solvents for the epoxidation reaction include low molecular weight alcohols such as isopropyl alcohol, glycol ethers such as Dowanol® PM, polar aprotic solvents such as dimethyl sulfoxide, chlorinated hydrocarbons, aliphatic and cycloaliphatic ethers and diethers, aromatic hydrocarbons, and mixtures thereof.

Although both the mixture of polyphenolic compounds and the epoxy resin of the present invention can be used alone, i.e., without the addition of any other resins (or may be used as a mixture of only the epoxy resin of the present invention and the mixture of polyphenolic compounds of the present invention) to make cured products, they will usually be used in combination with one or more resins which are different from the epoxy resin of the present invention and the mixture of polyphenolic compounds of the present invention. For example, the mixture of polyphenolic compounds and/or the epoxy resin of the present invention may be combined with other epoxy resins such as, e.g., diglycidyl ethers of bisphenol A or bisphenol F, and glycidyl ethers of phenol novolac or cresol novolac resins (i.e., glycidyl ethers of formaldehyde-based phenolic resins) in order to increase the thermal resistance and/or the toughness of corresponding cured products. Corresponding mixtures will often comprise from about 5 % to about 95% by weight, e.g., from about 10% to about 90%, from about 20% to about 80%, from about 30% to about 70%, or from about 40% to about 60% by weight of the mixture of polyphenolic compounds and/or the epoxy resin of the present invention, based on the total weight of the resin components.

The epoxy resins of the present invention can, for example, also be used in combination with a brominated bisphenol such as, e.g., tetrabromobisphenol A (TBBA), the diglycidyl ether of TBBA, or the oligomeric epoxy resins which are derived from TBBA and can be used for the manufacture of electrical laminates (e.g., FR4 electrical laminates). Non-brominated flame retardants such as phthalates (e.g., dioctyl phthalate), phosphates, phosphonates, and phosphinates, especially those derived from DOPO (6H-dibenz[c,e][1,2]oxaphosphorin-6-oxide) may also be used to yield a brominated epoxy resin or a halogen-free epoxy resin respectively, that can be used for the manufacture of electrical laminates (e.g., FR4 electrical laminates). Examples of typical hardeners for such formulations include dicyandiamide, polyphenols (such as, e.g., the mixture of polyphenolic compounds of the present invention), and anhydrides. Examples of solvents which may be used to make corresponding formulations include acetone, 2-butanone, cyclohexanone, methoxypropanols, and methoxypropanol acetate. Examples of other additives, catalyst and fillers which may be used include those which are conventionally employed.

The mixture of polyphenolic compounds of the present invention may be used in a similar fashion as the epoxy resin of the present invention by combining this mixture with, e.g., epoxy resins such as epoxy novolacs and the diglycidylether of a bisphenol such as bisphenol A. Additional hardeners as described above may be added, along with brominated and/or non-brominated flame retardants, for example, phthalates such as, e.g., dioctylphthalate to yield a halogen-free resin that can be used for the manufacture of electrical laminates (e.g., FR4 electrical laminates).

Other non-limiting examples of compounds and resins which may be combined (and co-cured) with the mixture of polyphenolic compounds and the epoxy resin of the present invention are disclosed in, e.g., the co-assigned applications entitled "AROMATIC DICYANATE COMPOUNDS WITH HIGH ALIPHATIC CARBON CONTENT" (Attorney Docket No. 66499), "AROMATIC POLYCYANATE COMPOUNDS AND PROCESS FOR THE PRODUCTION THEREOF" (Attorney Docket No. 66500) and "ETHYLENICALLY UNSATURATED MONOMERS COMPRISING ALIPHATIC AND AROMATIC MOIETIES" (Attorney Docket No. 66641), all filed concurrently herewith. The entire disclosures of these co-assigned applications are expressly incorporated by reference herein.

The curable mixtures of the present invention and the products made therefrom respectively, may further comprise one or more other substances such as, e.g., one or more additives which are commonly present in polymerizable mixtures and products made therefrom. Non-limiting examples of such additives include polymerization catalysts, co-curing agents, flame retardants, synergists for flame retardants, solvents, fillers, glass fibers, adhesion promoters, wetting aids, dispersing aids, surface modifiers, thermoplastic resins, and mold release agents.

Non-limiting examples of suitable curing agents and curing accelerators include, but are not limited to, amine-curing agents such as dicyandiamide, diaminodiphenylmethane and diaminodiphenylsulfone, polyamides, polyaminoamides, polyphenols, polymeric thiols, polycarboxylic acids and anhydrides such as phthalic anhydride, tetrahydrophthalic anhydride (THPA), methyl tetrahydrophthalic anhydride (MTHPA), hexahydrophthalic anhydride (HHPA), methyl hexahydrophthalic anhydride (MHHPA), nadic methyl anhydride (NMA), polyazealic polyanhydride, succinic anhydride, maleic anhydride and styrene-maleic anhydride copolymers, polyols, substituted or epoxy-modified imidazoles such as 2-methylimidazole, 2-phenyl imidazole and 2-ethyl-4-methyl imidazole, phenolic curing agents such as phenol novolac resins, tertiary amines such as triethylamine, tripropylamine and tributylamine, phosphonium salts such as ethyltriphenylphosphonium chloride, ethyltriphenylphosphonium bromide and ethyltriphenylphosphonium acetate, and ammonium salts such as benzyltrimethylammonium chloride and benzyltrimethylammonium hydroxide. Curing agents and accelerators are preferably used in total amounts of from about 0.5 % to about 20 % by weight, based on the total weight of the (curable) mixture (e.g., electrical laminate composition).

Non-limiting examples of flame retardants and synergists therefor for use in the present invention include phosphorus containing molecules such as adducts of DOPO (6H-dibenz[c,e][1,2]oxaphosphorin-6-oxide) with epoxy resins, especially epoxy novolacs, magnesium hydrate, zinc borate, and metallocenes. Brominated resins such as, e.g., tetrabromobisphenol A and the corresponding diglycidyl ether are another example of a flame retardant component which can be used in the curable mixtures of the present invention.

Non-limiting examples of solvents for use in the present invention (for example, for improving processability) include acetone, 2-butanone, and Dowanol® PM(A) (propylene glycol methyl ether (acetate) available from Dow Chemical Company).

Non-limiting examples of fillers for use in the present invention include functional and non-functional particulate fillers with a particle size range of from about 0.5 nm to about 100 µm. Specific examples thereof include silica, alumina trihydrate, aluminum oxide, metal oxides, carbon nanotubes, silver flake or powder, carbon black, and graphite.

Non-limiting examples of adhesion promoters for use in the present invention include modified organosilanes (epoxidized, methacryl, amino, allyl, etc.), acetylacetonates, sulfur containing molecules, titanates, and zirconates.

Non-limiting examples of wetting and dispersing aids for use in the present invention include modified organosilanes such as, e.g., Byk 900 series and W 9010, and modified fluorocarbons.

Non-limiting examples of surface modifiers for use in the present invention include slip and gloss additives, a number of which are available from Byk-Chemie, Germany.

Non-limiting examples of thermoplastic resins for use in the present invention include reactive and non-reactive thermoplastic resins such as, e.g., polyphenylsulfones, polysulfones, polyethersulfones, polyvinylidene fluoride, polyetherimides, polyphthalimides, polybenzimidazoles, acrylics, phenoxy resins, and polyurethanes.

Non-limiting examples of mold release agents for use in the present invention include waxes such as, e.g., carnauba wax.

The mixture of polyphenolic compounds as well as the epoxy resin of the present invention are useful, *inter alia*, as thermosettable components for the manufacture of electrical laminates (e.g., for printed circuit boards and materials for integrated circuit packaging such as IC substrates), for example, in order to increase the thermal resistance (e.g., thermal decomposition temperature > about 340°C) and/or the glass transition temperature (e.g., Tg > about 180°C) and/or to improve the toughness of corresponding cured products.

### Example 1

### A. Synthesis and Characterization of a Mixture of Polyphenolic Compounds Based on Cyclohexane Dicarboxaldehyde and Phenol

Phenol (598 g, 6.36 moles) and cyclohexane dicarboxaldehyde (74.2 g, 0.53 moles, mixture of 1,3- and 1,4-isomers; ratio of phenolic groups to aldehyde groups = 6 : 1, equivalent ratio of phenol to cyclohexane dicarboxaldehyde = 3 : 1) were added together in a 1-L 5-neck reactor. The mixture was heated to 50 °C with 500 rpm mechanical stirrer agitation. At 50 °C and atmospheric pressure, *p*-toluenesulfonic acid (PTSA) (1.3959 g total, 0.207% by weight) was added in six portions over 30 minutes. The temperature increased a few degrees with each PTSA addition. After the 6th PTSA addition, the temperature controller was set to 70 °C and vacuum was applied to the reactor. In order to avoid the reactor content flooding the rectifier, the reactor pressure was gradually decreased to remove water from the reaction solution. When the reflux had stopped, the reactor was vented and water (48 g) was added.

Water (79 g) and NaHCO₃ (0.6212 g) were added to neutralize the PTSA. When the reaction contents had cooled to room temperature, the entire contents were transferred to a 2-L separatory funnel. Methyl ethyl ketone (MEK) was added, and the contents were washed several times with water to remove PTSA-salt. The solvents and excess phenol were removed using a rotary evaporator, and the hot novolac was poured onto aluminum foil. The reaction of phenol with cyclohexane dicarboxaldehyde produced as the predominant product a tetraphenol possessing the following idealized structure:

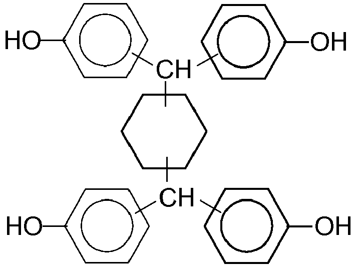

Ultraviolet spectrophotometric analysis provided a hydroxyl equivalent weight (HEW) of 118.64. High pressure liquid chromatographic (HPLC) analysis was adjusted to resolve 24 (isomeric) components present in the product.

### Example 2

### Epoxidation of Mixture of Polyphenolic Compounds

The mixture of polyphenolic compounds obtained according to Example 1 (107.5 g, 0.22 moles based on the assumption of 100 % tetraphenol of the above structure), epichlorohydrin (414.08 g, 4.51 moles; ratio of epoxy groups to phenolic groups = equivalent ratio of epichlorohydrin to polyphenolic compounds = about 5.1 : 1) and Dowanol® PM (propylene glycol methyl ether available from Dow Chemical Company; 79.4 g, 12.7 % by weight) were added to a 1.5 L 5-neck reactor. The resultant solution was heated to 65°C while being agitated at 650 rpm. Upon reaching 65°C, vacuum (285 mbar) was applied to the reactor and 50 % aqueous NaOH (72.9 g) was added to the reaction mixture over a period of 4 hours. Upon completion of the addition, the reaction mixture was kept for an extra 15 minutes at 65°C and then the entire reactor content was filtered to remove by-product salt. The filtrate was transferred to a 2-L separatory funnel, methyl ethyl ketone was added to the filtrate and the resultant mixture was extracted several times with water to remove residual salt. Thereafter, the washed solution was concentrated on a rotary evaporator to remove unreacted epichlorohydrin. The resultant neat resin was poured onto aluminum foil.

### Example 3

The preparation of a mixture of polyphenolic compounds from cyclohexane dicarboxaldehyde and phenol and the epoxidation of this mixture with epichlorohydrin were carried out in a 1-L flask under several different reaction conditions. The reaction conditions and the properties of the resultant products are summarized in Table 1 below.

**Table 1**

| **Processing Condition or Property** | **Run 1** | **Run 2** | **Run 3** | **Run 4** | **Run 5** |
|---|---|---|---|---|---|
| Phenol/CHDA equivalent Ratio | 3/1 | 3/1 | 3/1 | 3/1 | 5/1 |
| PTSA catalyst, wt. % | 0.254 | 0.246 | 0.188 | 0.2 | 0.2 |
| Reaction Temp, deg C | 80 | 80 | 80 | 65 | 60 |
| Reaction Time, min | 180 | 300 | 240 | 265 | 265 |
| | | | | | |
| Epi/polyphenolic equivalent Ratio | 2.5/1 | 2.5/1 | 2.5/1 | 5/1 | 5/1 |
| NaOH Equivalents | 1.02 | 1.02 | 1.02 | 1.02 | 1.02 |
| Reaction Temp., deg C | 65 | 65 | 65 | 65 | 65 |
| Reaction Time, min. | 263 | 257 | 247 | 249 | 257 |
| | | | | | |
| Novolac Monomer, LC area % | 72.19 | 74.31 | 71.5 | 84.46 | 78.75 |
| Novolac, Mn | 767 | 738 | | | |
| Epoxy, Mn | 918 | 924 | | | |
| % Epoxide | 21.83 | 21.28 | 21.26 | 22.47 | 22.57 |
| EEW | 197 | 202 | 202 | 191 | 191 |
| Viscosity, cts | 7695 | 9644 | 12078 | 4111 | 1130 |
| HyCl, ppm | 99 | 47 | 42 | 39 | 49 |
| Total Chloride, ppm | 1400 | 1175 | 1243 | 1256 | 1185 |
| Metier Softening Pt.-Epoxy, deg C | 140 | 145 | 145 | 141 | 122 |

| | | | | | |
|---|---|---|---|---|---|
| CHDA = cyclohexane dicarboxaldehyde Epi = epichlorohydrin PTSA = *p*-toluenesulfonic acid LC = liquid chromatography EEW = epoxy equivalent weight (molecular weight per epoxy group) | | | | | |

The percentage of tetraphenolic compound (= compound of the idealized formula depicted in Example 1 above) was estimated based on liquid chromatography-mass spectrometric analysis and gel permeation chromatographic analysis. These analyses revealed as many as 11 species of polyphenolic compound. The large number of isomers is considered to be the result of the combined isomeric species of the dicarboxaldehyde and phenol.

### Example 4

One of the proposed uses of the epoxy resins of the present invention is that as an additive to a high Tg brominated epoxy laminate system to further boost the Tg and the thermal decomposition temperature (Td) of the laminate. To investigate how an epoxy resin of the present invention would behave in a laminate system, a comparative study was performed using a commercially available tri-functional epoxidized novolac resin additive which is known to improve Tg/Td (EPPN501H available from Nippon Kayaku). The comparative study was performed using a base system comprising D.E.N.™ 438 (epoxidized phenol-formaldeyde novolac resin, average functionality 3.6, epoxy equivalent weight 176-181, available from Dow Chemical Company), D.E.R.™ 542 (diglycidyl ether of tetrabromobisphenol A available from Dow Chemical Company; bromine source which was kept at 42 % by weight to maintain a constant level of 20 % by weight of bromine for fire resistance), 2-ethyl-4-methylimidazole as catalyst, DURITE™ SD1731 (a phenolic novolac curing agent available from Borden Industrial Products, Louisville, Ky) and either a cyclohexane dicarboxaldehyde epoxy resin (CHDAE) according to the present invention (the product of Run 1 of the above Example 3) or EPPN501H as the performance enhancing additive. The variables in the study were the weight ratio of the D.E.N.™ 438 to the performance enhancing additive and the amount of catalyst. The reaction conditions and results are summarized in the following table.

The test samples were prepared as follows: The phenolic and epoxy components were mixed in the presence of catalyst and solvent (e.g., acetone, 2-butanone, Dowanol® PM, Dowanol®PMA, etc.) to make a solution having a solids content of about 60 - 65 % by weight. The solution was placed in a closed glass container and agitated at room temperature for 1 day in an ultrasonic bath. A portion of the solution was then placed on a hot plate at about 171°C for approximately 5 minutes until substantially all of the solvent had been removed and the mixture was cured. The residue on the hot plate was then removed and placed in an oven at about 190°C for about 1 hour to allow the mixture to fully cure. This material was the used as the sample for the DSC analysis (Tg) and the TGA analysis (Td). The DMA analysis used solution as described in the test method.

### Test Methods:

### Differential Scanning Calorimetry (DSC) for determining Tg

DSC was carried out on an instrument 2929 DSC (TA Instruments) using IPC Method 2.4.24. Two scans were made on the same sample at 20°C/min. with a cool-down period (15 minutes at 190°C) in-between runs. The reported Tg values are the midpoint of the transition region in the second scan.

### Dynamic Mechanical Analysis (DMA) for determining Tg and modulus

Thin film samples were prepared by coating a tin-free steel panel using a draw down bar and then curing at 190°C for 2 hours. The films were removed using mercury amalgam. The films were then subjected to DMA on an instrument RSA II (TA Instruments). The samples were run in the tension-tension mode at 1 Hz from room temperature to 275°C at 5°C/min. Some samples were subjected to a second scan to check for complete cure. All samples were confirmed to be fully cured.

### Thermogravimetric Analysis (TGA) for determining Td

The thermal decomposition temperature (Td) was measured by a TGA instrument from TA Instruments under a nitrogen atmosphere using IPC Method 2.4.24.6. Samples were heated from 25°C up to 450°C at a heating rate of 10°C/min. The temperature at which the laminate underwent 5% weight loss was recorded as the Td.

Results and reaction conditions are summarized in Table 2 below.

**Table 2**

| 2E4MI | 438:EPPN or CHDAE | Tg by DSC | | Tg by DMA | | Td | | E' Rubbery Modulus [xE+8 dynes/cm^2] | | |
|---|---|---|---|---|---|---|---|---|---|---|
| | | CHDAE | EPPN | CHDAE | EPPN | CHDAE | EPPN | CHDAE | EPPN | |
| 0.1 | 0.875 | 186 | 184 | 193 | 188 | 344 | 347 | 4.8 | 6.16 | |
| 0.05 | 0.250 | 191 | 187 | 205 | 191 | 356 | 354 | 4.1 | 6.29 | |
| 0.15 | 1.500 | 183 | 184 | 188 | 186 | 346 | 345 | 3.2 | 5.50 | |
| 0.1 | 0.875 | 183 | 183 | 193 | 186 | 353 | 348 | 3.5 | 5.71 | |
| 0.05 | 1.500 | 179 | 178 | 181 | 176 | 356 | 356 | 4 | 4.70 | |
| 0.15 | 0.250 | 190 | 191 | 201 | 196 | 348 | 345 | 5.1 | 5.76 | |
| 0.1 | 0.875 | 183 | 183 | 193 | 187 | 352 | 349 | 3.9 | 5.70 | |

| | | | | | | | | | | |
|---|---|---|---|---|---|---|---|---|---|---|
| 2E4MI = 2-ethyl-4-methylimidazole | | | | | | | | | | |

As can be seen from the above results, in almost all cases the additive according to the present invention afforded a higher Tg by DMA and a higher Td than the comparative additive. However, what is most remarkable is that the additive of the present invention proved to be significantly superior to the comparative additive with respect to an improvement of the potential thoughness of the system, as indicated by a significantly lower rubbery modulus (>Tg) in all cases.

### Example 5

Another comparative study was performed. The resin components used are shown in Table 3, below. CHTP stands for cyclohexane tetraphenol and eCHTP is the epoxy of cyclohexane tetraphenol. BPAN is a bisphenol A novolac and eBPAN is the epoxy of bisphenol A novolac. Rezicure 3026 is a phenolic novoloc from S1 group. 2-MI is 2-methylimidazole.

**Table 3**

| *Components* | EW | % *solids* | *Wt. (g)* | *Wt.* % | *Solution Wt.(g)* | *Actual Wt (g)* |
|---|---|---|---|---|---|---|
| | | | | | | |
| *eCHTP* | *195* | 100 | *1283.12* | *34.75* | *1283.12* | *1284.00* |
| *D.E.R.™ 560* (*70% NV in 70:30 DOWANOL™ PMA*/*Acetone*) | 450 | 70 | *1279.34* | *34.65* | *1827.62* | *1827.70* |
| *CHTP* (*60%NV in50:50 MEK*/*DOWANOL*™ *PM)* | 120 | 60 | 1129.92 | *30.60* | *1883.19* | *1884.10* |
| *2-MI* (*20% NV in MeOH*) | | 20 | *1.1037* | *0.030* | *5.5184* | *5.2000* |
| | | | | | | |
| *eBPAN* | 218 | 100 | *1368.43* | *36.70* | *1368.43* | *1368.50* |
| D.E.R. ™560 (*70% NV in 70:30 DOWANOL*™ *PMA*/*Acetone*) | 450 | 70 | *1290.04* | *34.60* | *1842.91* | *1842.93* |
| *BPAN (60% NV in 50:50 MEK*/*Dowanol*™*PM*) | *117* | 60 | *1069.93* | *28.70* | *1783.21* | *1784.10* |
| *2-MI (20% NV in MeOH*) | | *20* | *1.1121* | *0.030* | *5.5604* | *5.3000* |
| | | | | | | |
| *D.E.N.*™ *438* | *180* | *85* | *1206.45* | *36.30* | *1419.35* | *1420.00* |
| *D.E.R.*™ *560* (*70% NV in 70:30 DOWANOL*™ *PMA*/*Acetone*) | *450* | *70* | *1153.03* | *34.69* | *1647.19* | *1649.00* |
| *Rezicure® 3026* (50% *NV in 50:50 DOWANOL*™ *PM*/*MEK)* | *104* | *50* | *964.08* | *29.01* | *1928.15* | *1930.00* |
| *2-MI (20% NV in MeOH)* | | *20* | *0.9838* | *0.030* | *4.9188* | *6.8000* |

The properties of each formulation are shown in Table 4, below. CTE is the coefficient of thermal expansion. The CHTP formulation has a Tg of about 30°C higher or more than the non-CHTP formulations.

**Table 4**

| | DEN™438/R3026 | eBPAN/BPAN | eCHTP/CHTP |
|---|---|---|---|
| Laminate Thickness (mm) | 1.40-1.60 | 1.42-1.65 | 1.5-1.73 |
| Tg1 (deg C, DSC) | 155 | 181 | **212** |
| Tg2 (deg C, DSC) | 159 | 185 | **214** |
| Tg3 (deg C, DSC) | 164 | 187 | **218** |
| Td (5% wt loss) | 360 | 368 | 367 |
| % resin | 43 | 45 | 50 |
| T288(min) | 26 | >30 | 23 |
| CTE<Tg (ppm/deg C) | 59 | 47 | 86 |
| CTE>Tg (ppm/deg C) | 269 | 224 | 222 |
| Cu Peel (lb/in) | 6.9956 | 6.3364 | 6.0376 |
| Water Uptake (%) | 0.256 | 0.252 | 0.354 |

| | | | |
|---|---|---|---|
| Td - thermal decomposition T-288 - time to delamination at 288 deg C | | | |

### Example 6

A Fusion-Bonded Epoxy (FBE) powder coating formulation was prepared by compounding 672.2 g of D.E.R.™ 664UE (available from the Dow Chemical Company, a "4-type" solid diglycidyl ether of bisphenol A having an epoxy equivalent weight of 860-930 and a softening point of 104-110°C), 9.3 g of Amicure^{®} CG 1200 (dicyandiamide powder available from Air Products), 5.0 g of Epicure^{™} P 101 (2-methylimidazole adduct with bisphenol A epoxy resin available from Shell Chemical), 10 g of Modaflow^{®} Powder III (flow modifier, ethyl acrylate/2-ethylhexylacrylate copolymer in silica carrier manufactured by UCB Surface Specialties of St. Louis, Mo), 303.4 g of Vansil^{®} W 20 (wollastonite filler available from The Cary Company of Addison, IL) and 3.0 g of Cab-O-Sil^{®} M 5 (colloidal silica available from Cabot Corp.). A steel bar heated at 242°C was immersed into the resulting coating powder, then allowed to cure for 2 min at 242°C and water quenched for 10 minutes. The resulting Fusion-Bonded Epoxy coating showed an onset Tg of 104°C and a good adhesion to the steel substrate.

### Example 7

A Fusion-Bonded Epoxy powder coating formulation was prepared by compounding 754.8 g of XZ 92457.02 (isocyanate modified epoxy resin made from bisphenol A, epichlorohydrin and methylenediphenylene diisocyanate, commercially available from the Dow Chemical Company, CAS No. 60684-77-7), 22.2 g of Amicure^{®} CG 1200 (dicyandiamide powder available from Air Products), 11.2 g of Epicure^{™} P 101 (2-methylimidazole adduct with bisphenol A epoxy resin available from Shell Chemical), 13 g of Curezol^{®} 2PHZ-PW (imidazole epoxy hardener available from Shikoku), 5 g of Modaflow^{®} Powder III (flow modifier, ethyl acrylate/2-ethylhexylacrylate copolymer in silica carrier manufactured by UCB Surface Specialties of St. Louis, Mo), 193.8 g of Minspar^{™} 7 (feldspar filler) and 3.0 g of Cab-O-Sil^{®} M 5 (colloidal silica available from Cabot Corp.). A steel bar heated at 242°C was immersed into the resulting coating powder, then allowed to cure for 2 min at 242°C and water quenched for 10 minutes. The resulting Fusion-Bonded Epoxy coating showed an onset Tg of 160°C and a good adhesion to the steel substrate.

### Examples 8 - 11

In a manner similar to that described in Examples 5 and 6 FBE powder coating formulations were prepared from the components listed in Table 5 below.

### Film Preparation for Testing

Void-free thin films of the formulations prepared in Examples 6-11 above were made for Differential Scanning Calorimetry (DSC), Thermo-Gravimetric Analysis (TGA) and tensile testing. The free films were prepared by attaching a 75 mm by 150 mm sheet of DuoFoil onto a steel panel (3 by 75 by 200 mm), pre-heating this panel in a Blue M convection oven set at 242°C for 30 minutes, then placing it in a fluidized bed containing the powder coatings. The coated panel was then immediately placed in an oven at 242°C for 2 minutes to cure the coating. After curing, the panel was quenched in a water bath at ambient temperature for 2 minutes. The FBE coating film was then removed from the DuoFoil.

### Differential Scanning Calorimetry (DSC)

10-20 mg samples were cut from the film samples with a razor blade and placed into open aluminum pans. The pans were crimped, then subjected to a dynamic temperature scan under nitrogen from room temperature to 260°C at 20°C/min using a TA Model Q1000 DSC instrument. The Tg's from the first scan and the second scan were recorded. Test results for the films made from the formulations of Examples 6-11 are summarized in Table 5 below.

### Thermo-Gravimetric analysis (TGA)

TGA samples (~5 mg) were chipped from film samples. Weight loss was monitored using a TA Instruments Q5000 TGA using a temperature ramp from room temperature to 750°C in air. The Thermal Decomposition temperature was measure at 5% weight loss. Test results for the films made from the formulations of Examples 6-11 are summarized in Table 5 below.

### Tensile properties measurement

Microtensile tests were performed on dog bone shaped thin film samples using an MTS Alliance RT-10 instrument. The dog bone samples were stamped from rectangular films to dimensions of approximately 38 mm x 5 mm x 0.254 mm using a microtensile die in a manual press. The tests (based on ASTM D638) were conducted at room temperature at an extension rate of 0.03 mm/sec. Load and displacement data were used to calculate the tensile modulus, tensile strength, and tensile strain at break. Test results for the films made from the formulations of Examples 6-11 are summarized in Table 5 below.

**Table 5**

| **Example No.** | **6*** | **7*** | **8*** | **9*** | **10** | **11** |
|---|---|---|---|---|---|---|
| **D.E.R.™ 664UE, g** | 672.2 | | 528 | | 597 | |
| **XZ 92457.02, g** | | 754.8 | | 411.3 | | 508.5 |
| **Amicure^{®} CG 1200, g** | 9.3 | 22.2 | | | | |
| **D.E.H.™ 85, g⁽¹⁾** | | | 141.5 | 230.9 | | |
| **CHTP, g⁽²⁾** | | | | | 75.4 | 129.4 |
| **EPI-CURE™ P101, g** | 5.0 | 11.2 | 10 | 2.4 | 10 | 3.03 |
| **CUREZOL^{®} 2PHZ 7/10, g** | | 13.0 | | 2.8 | | 3.51 |
| **Modaflow^{®} powder III, g** | 10.0 | 5.0 | 10.1 | 4.0 | 10 | 3.99 |
| **Vansil^{®} W 20, g** | 303.4 | | 310.6 | | 307.2 | |
| **Minspar™ 7, g** | | 193.8 | | 148.8 | | 151.8 |

| **Properties** | | | | | | |
|---|---|---|---|---|---|---|
| **Gel Time (sec)** | 39 | 27.8 | 39 | 65.1 | 31 | 29.5 |
| **Enthalpy (J/g)** | 77.7 | 151.4 | 51 | 73.6 | 44.0 | 74.6 |
| **Tg1 (°C) of the powder coating** | 56.3 | 53.6 | 53 | 55.3 | 68 | 64.9 |
| **Tg2 (°C) of the powder coating** | 104.1 | 160.2 | 99 | 123.8 | 112 | 156.6 |
| **Tensile Strength (psi)** | | 4045.9 | | 5569.1 | | 6861.3 |
| **Tensile Modulus (psi)** | | 181327.9 | | 305642.7 | | 347701.4 |
| **Elongation at Break (%)** | | 3.8 | | 4.2 | | 4.5 |
| **Tg (°C) of the free film** | | 161.7 | | 122.7 | | 162.7 |
| **Temp @ 5 wt% Loss (°C)** | | 332.4 | | 399.7 | | 392.5 |

| | | | | | | |
|---|---|---|---|---|---|---|
| * = Comparative Example (1) Phenolic epoxy hardener available from the Dow Chemical Company. Based on an unmodified solid reaction product of liquid epoxy resin and bisphenol A and having an epoxy equivalent weight of 250-280. (2) Product from Example 1 | | | | | | |

As can be seen from the results summarized in Table 5, compared to a dicyandiamide hardener (Amicure^{®} CG 1200) and a conventional phenolic hardener (D.E.H.^{™} 85), in combination with both an epoxy resin and a modified epoxy resin the mixture of polyphenolic compounds of the present invention (CHTP) affords FBE films with a superior combination of thermal resistance (Temp @ 5 wt% Loss), Tg and tensile properties.

Although the present invention has been described in considerable detail with regard to certain versions thereof, other versions are possible, and alterations, permutations, and equivalents of the version shown will become apparent to those skilled in the art upon a reading of the specification and study of the drawings. Also, the various features of the versions herein can be combined in various ways to provide additional versions of the present invention. Furthermore, certain terminology has been used for the purposes of descriptive clarity, and not to limit the present invention. Therefore, any appended claims should not be limited to the description of the preferred versions contained herein and should include all such alterations, permutations, and equivalents as fall within the true spirit and scope of the present invention.

Having now fully described this invention, it will be understood to those of ordinary skill in the art that the methods of the present invention can be carried out with a wide and equivalent range of conditions, formulations, and other parameters without departing from the scope of the invention or any embodiments thereof.

## Claims

1. A mixture which comprises
(i) at least one of (a) the mixture of polyphenolic compounds and/or a prepolymerized form thereof and (b) an epoxy resin and/or a prepolymerized form thereof and
(ii) at least one of (c) a novolac resin and (d) an epoxy resin which is different from (b);
wherein the mixture of polyphenolic compounds (a) is obtainable by a process comprising: condensing a dialdehyde of a cycloalkane having from about 5 to about 24 ring carbon atoms with a phenolic compound at a ratio of phenolic hydroxy groups to aldehyde groups which affords a mixture of polyphenolic compounds which comprises at least about 20 % by weight of a polyphenolic compound of formula (I): 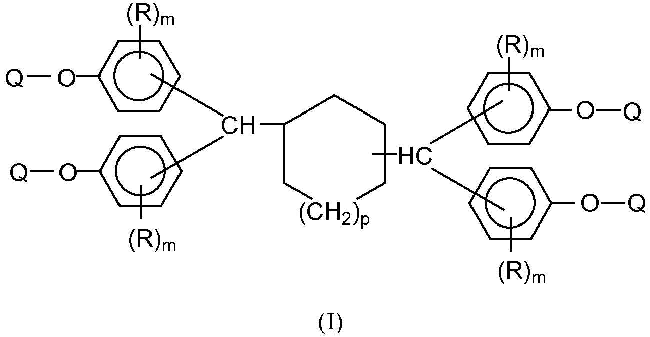 wherein:
p is 0 or an integer of from 1 to about 19;
each m independently is 0, 1, or 2;
the moieties R independently represent halogen, cyano, nitro, hydroxy, optionally substituted alkyl, optionally substituted cycloalkyl, optionally substituted alkoxy,
optionally substituted alkenyl, optionally substituted alkenyloxy, optionally substituted aryl, optionally substituted aralkyl, optionally substituted aryloxy, and optionally substituted aralkyloxy; and
the moieties Q represent hydrogen;
and any non-aromatic cyclic moieties comprised in the above formula (I) may optionally carry one or more substituents and/or may optionally comprise one or more double bonds;
and wherein epoxy resin (b) is obtained by partially or completely converting phenolic hydroxy groups of a mixture of polyphenolic compounds (a) into glycidyl ether groups.

2. The mixture of claim 1, wherein the mixture of polyphenolic compounds comprises at least about 50 % by weight of the polyphenolic compound of formula (I).

3. The mixture of any one of claims 1 and 2, wherein a molar ratio of phenolic compound to cycloalkane dialdehyde in the process is at least about 5 : 1.

4. The mixture of any one of claims 1 to 3, wherein the cycloalkane has 6, 7 or 8 ring carbon atoms.

5. The mixture of any one of claims 1 to 4, wherein the dialdehyde in the process comprises a cyclohexane dicarboxaldehyde.

6. The mixture of any one of claims 1 to 5, wherein the phenolic compound in the process comprises phenol.

7. The mixture of any one of claims 1 to 6, wherein the epoxy resin (b) is obtained by converting substantially all of the phenolic hydroxy groups into glycidyl ether groups.

8. The mixture any one of claims 1 to 7, wherein the epoxy resin (d) comprises an epoxidized novolac resin.

9. The mixture of any one of claims 1 to 8, wherein the mixture comprises a brominated epoxy resin.

10. The mixture of any one of claims 1 to 9, wherein the mixture further comprises one or more substances which are selected from polymerization catalysts, co-curing agents, flame retardants, synergists for flame retardants, solvents, fillers, glass fibers, adhesion promoters, wetting aids, dispersing aids, surface modifiers, thermoplastic polymers, and mold release agents.

11. The mixture of any one of claims 1 to 10, wherein the mixture is partially or completely cured.

12. A product which comprises the mixture of any one of claims 1 to 11.
A

13. The product of claim 12, wherein the product is at least one of an electrical laminate, an IC substrate, a casting, a coating, a die attach and mold compound formulation, a composite, a potting composition, and an adhesive.

14. A method of making the mixture of any one of claims 1 to 11.

15. Use of at least one of (a) a mixture of polyphenolic compounds and/or a prepolymerized form thereof and (b) an epoxy resin and/or a prepolymerized form thereof for increasing at least one of the thermal resistance and the toughness of a resin material;
wherein the mixture of polyphenolic compounds (a) is obtainable by a process comprising: condensing a dialdehyde of a cycloalkane having from about 5 to about 24 ring carbon atoms with a phenolic compound at a ratio of phenolic hydroxy groups to aldehyde groups which affords a mixture of polyphenolic compounds which comprises at least about 20 % by weight of a polyphenolic compound of formula (I): 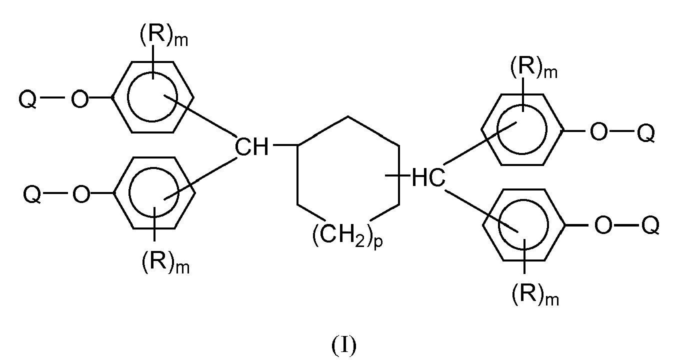 wherein:
p is 0 or an integer of from 1 to about 19;
each m independently is 0, 1, or 2;
the moieties R independently represent halogen, cyano, nitro, hydroxy, optionally substituted alkyl, optionally substituted cycloalkyl, optionally substituted alkoxy,
optionally substituted alkenyl, optionally substituted alkenyloxy, optionally substituted aryl, optionally substituted aralkyl, optionally substituted aryloxy, and
optionally substituted aralkyloxy; and
the moieties Q represent hydrogen;
and any non-aromatic cyclic moieties comprised in the above formula (I) may optionally carry one or more substituents and/or may optionally comprise one or more double bonds; and
wherein epoxy resin (b) is obtained by partially or completely converting phenolic hydroxy groups of a mixture of polyphenolic compounds (a) into glycidyl ether groups; and
wherein the resin material is made from at least one of (c) a novolac resin and (d) an epoxy resin which is different from (b).

16. Use according to claims 15 wherein the material which results from the method comprises 5% to 95% by weight of the mixture of polyphenolic compounds and/or the epoxy resin of the present invention, based on the total weight of the resin components.

17. Use according to any one of claims 15 to 16 wherein p is 1.

18. Use according to any one of claims 15 to 17 wherein the epoxy resin (d) comprises an epoxidized novolac resin.

19. An epoxy resin which is obtainable by a process comprising partially or completely converting phenolic hydroxy groups of the mixture of polyphenolic compounds into glycidyl ether groups;
wherein the mixture of polyphenolic compounds is obtainable by a process comprising condensing a dialdehyde of a cycloalkane having 6 ring carbon atoms with a phenolic compound at a ratio of phenolic hydroxy groups to aldehyde groups which affords a mixture of polyphenolic compounds which comprises at least about 20 % by weight of a polyphenolic compound of formula (I): 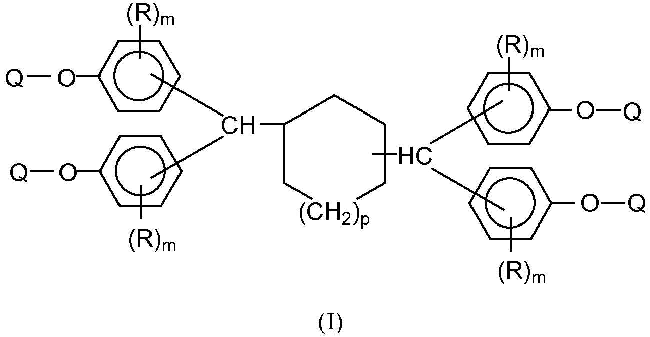 wherein:
p is 1;
each m independently is 0, 1, or 2;
the moieties R independently represent halogen, cyano, nitro, hydroxy, optionally substituted alkyl, optionally substituted cycloalkyl, optionally substituted alkoxy,
optionally substituted alkenyl, optionally substituted alkenyloxy, optionally substituted aryl, optionally substituted aralkyl, optionally substituted aryloxy, and optionally substituted aralkyloxy; and
the moieties Q represent hydrogen;
and any non-aromatic cyclic moieties comprised in the above formula (I) may optionally carry one or more substituents and/or may optionally comprise one or more double bonds.

20. An epoxy resin according to claim 19, wherein the mixture of polyphenolic compounds is obtainable by a process comprising condensing a dialdehyde of a cycloalkane having 6 ring carbon atoms with a phenolic compound which comprises phenol.

21. An epoxy resin according to claim 19 or claim 20, which is obtainable by a process comprising contacting the mixture of polyphenolic compounds with epichlorohydrin.

22. An epoxy resin according to any one of claims 19 to 21, which is obtainable by a process wherein substantially all of the phenolic hydroxy groups are converted into glycidyl ether groups.

## Patentansprüche

1. Mischung, umfassend:
(i) mindestens eines von (a) der Mischung aus polyphenolischen Verbindungen und/oder einer vorpolymerisierten Form davon und (b) einem Epoxidharz und/oder einer vorpolymerisierten Form davon und
(ii) mindestens eines von (c) einem Novolakharz und (d) einem Epoxidharz, das von (b) verschieden ist,
wobei die Mischung aus polyphenolischen Verbindungen (a) erhältlich ist durch ein Verfahren, das umfasst: Kondensieren eines Dialdehyds eines Cycloalkans mit etwa 5 bis etwa 24 Ringkohlenstoffatomen mit einer phenolischen Verbindung bei einem Verhältnis von phenolischen Hydroxygruppen zu Aldehydgruppen, das eine Mischung aus polyphenolischen Verbindungen liefert, die mindestens etwa 20 Gew.-% einer polyphenolischen Verbindung mit der Formel (I) umfasst: 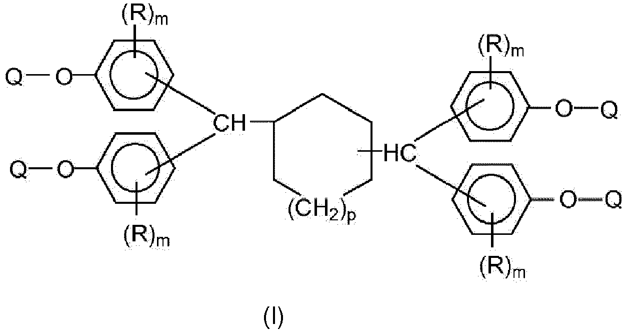 wobei
p gleich 0 ist oder eine ganze Zahl von 1 bis etwa 19 ist,
jedes m unabhängig 0, 1 oder 2 ist,
die Reste R unabhängig Halogen, Cyan, Nitro, Hydroxy, optional substituiertes Alkyl, optional substituiertes Cycloalkyl, optional substituiertes Alkoxy, optional substituiertes Alkenyl, optional substituiertes Alkenyloxy, optional substituiertes Aryl, optional substituiertes Aralkyl, optional substituiertes Aryloxy und optional substituiertes Aralkyloxy darstellen, und
die Reste Q Wasserstoff darstellen,
und alle in der obenstehenden Formel (I) umfassten nichtaromatischen cyclischen Reste optional einen oder mehr Substituenten tragen können und/oder optional eine oder mehrere Doppelbindungen aufweisen können,
und wobei das Epoxidharz (b) erhalten ist durch teilweises oder vollständiges Umwandeln phenolischer Hydroxygruppen einer Mischung aus polyphenolischen Verbindungen (a) in Glycidylethergruppen.

2. Mischung nach Anspruch 1, wobei die Mischung aus polyphenolischen Verbindungen mindestens etwa 50 Gew.-% der polyphenolischen Verbindung der Formel (I) aufweist.

3. Mischung nach einem der Ansprüche 1 und 2, wobei ein Molverhältnis von phenolischen Verbindungen zu Cycloalkandialdehyd in dem Verfahren bei mindestens etwa 5:1 liegt.

4. Mischung nach einem der Ansprüche 1 bis 3, wobei das Cycloalkan 6, 7 oder 8 Ringkohlenstoffatome besitzt.

5. Mischung nach einem der Ansprüche 1 bis 4, wobei das Dialdehyd in dem Verfahren einen Cyclohexandicarboxaldehyd umfasst.

6. Mischung nach einem der Ansprüche 1 bis 5, wobei die phenolische Verbindung in dem Verfahren Phenol umfasst.

7. Mischung nach einem der Ansprüche 1 bis 6, wobei das Epoxidharz (b) erhalten ist durch Umwandeln von im Wesentlichen allen phenolischen Hydroxygruppen in Glycidylethergruppen.

8. Mischung nach einem der Ansprüche 1 bis 7, wobei das Epoxidharz (d) ein epoxidiertes Novolakharz umfasst.

9. Mischung nach einem der Ansprüche 1 bis 8, wobei die Mischung ein bromiertes Epoxidharz umfasst.

10. Mischung nach einem der Ansprüche 1 bis 9, wobei die Mischung ferner eine oder mehrere Substanzen umfasst, die ausgewählt sind aus Polymerisationskatalysatoren, Co-Härtungsmitteln, Flammschutzmitteln, Flammschutzmittelsynergisten, Lösungsmitteln, Füllstoffen, Glasfasern, Adhäsionsverbesserern, Benetzungshelfern, Dispergiermitteln, Oberflächenmodifizierern, thermoplastischen Polymeren und Formtrennmitteln.

11. Mischung nach einem der Ansprüche 1 bis 10, wobei die Mischung teilweise oder vollständig ausgehärtet ist.

12. Produkt, welches die Mischung nach einem der Ansprüche 1 bis 11 umfasst.

13. Produkt nach Anspruch 12, wobei das Produkt mindestens eines von einem elektrischen Laminat, einem IC-Substrat, einem Gussteil, einer Beschichtung, einer Die-Attach- und Form-Verbindungsformulierung, einem Verbundwerkstoff, einer Vergusszusammensetzung und einem Klebstoff ist.

14. Verfahren zur Herstellung der Mischung nach einem der Ansprüche 1 bis 11.

15. Verwendung mindestens eines von (a) einer Mischung aus polyphenolischen Verbindungen und/oder einer vorpolymerisierten Form davon und (b) einem Epoxidharz und/oder einer vorpolymerisierten Form davon zum Erhöhen mindestens eines der Hitzebeständigkeit und der Zähigkeit eines Harzmaterials,
wobei die Mischung aus polyphenolischen Verbindungen (a) erhältlich ist durch ein Verfahren, welches umfasst: Kondensieren eines Dialdehyds eines Cycloalkans mit etwa 5 bis etwa 24 Ringkohlenstoffatomen mit einer phenolischen Verbindung bei einem Verhältnis von phenolischen Hydroxygruppen zu Aldehydgruppen, das eine Mischung von polyphenolischen Verbindungen liefert, die zumindest etwa 20 Gew.-% einer polyphenolischen Verbindung der Formel (I) aufweist: 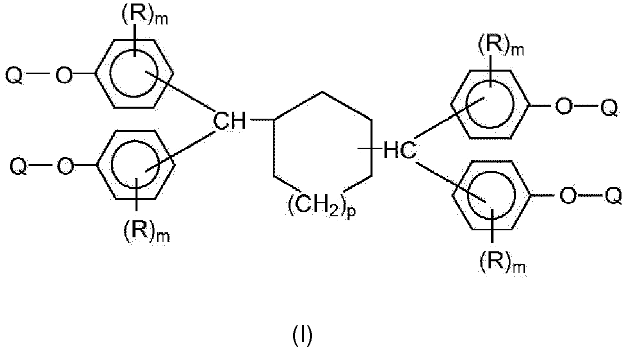 wobei
p gleich 0 ist oder eine ganze Zahl von 1 bis etwa 19 ist,
jedes m unabhängig 0, 1 oder 2 ist,
die Reste R unabhängig Halogen, Cyan, Nitro, Hydroxy, optional substituiertes Alkyl, optional substituiertes Cycloalkyl, optional substituiertes Alkoxy, optional substituiertes Alkenyl, optional substituiertes Alkenyloxy, optional substituiertes Aryl, optional substituiertes Aralkyl, optional substituiertes Aryloxy und optional substituiertes Aralkyloxy darstellen, und
die Reste Q Wasserstoff darstellen,
und alle in der obenstehenden Formel (I) umfassten nichtaromatischen cyclischen Reste optional einen oder mehrere Substituenten tragen können und/oder optional eine oder mehrere Doppelbindungen aufweisen können, und
wobei das Epoxidharz (b) erhalten ist durch teilweises oder vollständiges Umwandeln phenolischer Hydroxygruppen einer Mischung aus polyphenolischen Verbindungen (a) in Glycidylethergruppen, und
wobei das Harzmaterial hergestellt ist aus mindestens einem von (c) einem Novolakharz und (d) einem Epoxidharz, welches von (b) verschieden ist.

16. Verwendung nach Anspruch 15, wobei das aus dem Verfahren erhaltene Material 5 bis 95 Gew.-% der Mischung aus polyphenolischen Verbindungen und/oder des erfindungsgemäßen Epoxidharzes umfasst, bezogen auf das Gesamtgewicht der Harzbestandteile.

17. Verwendung nach einem der Ansprüche 15 bis 16, wobei p gleich 1 ist.

18. Verwendung nach einem der Ansprüche 15 bis 17, wobei das Epoxidharz (d) ein epoxidiertes Novolakharz umfasst.

19. Epoxidharz, das erhältlich ist durch ein Verfahren, umfassend teilweises oder vollständiges Umwandeln phenolischer Hydroxygruppen der Mischung aus polyphenolischen Verbindungen in Glycidylethergruppen,
wobei die Mischung aus polyphenolischen Verbindungen erhältlich ist durch ein Verfahren, umfassend das Kondensieren eines Dialdehyds eines Cycloalkans mit 6 Ringkohlenstoffatomen mit einer phenolischen Verbindung bei einem Verhältnis von phenolischen Hydroxygruppen zu Aldehydgruppen, das eine Mischung von polyphenolischen Verbindungen liefert, die mindestens etwa 20 Gew.-% einer polyphenolischen Verbindung der Formel (I) umfasst: 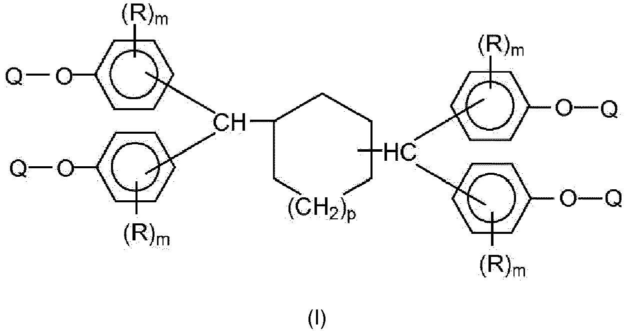 wobei
p gleich 1 ist,
jedes m unabhängig 0, 1 oder 2 ist,
die Reste R unabhängig Halogen, Cyan, Nitro, Hydroxy, optional substituiertes Alkyl, optional substituiertes Cycloalkyl, optional substituiertes Alkoxy, optional substituiertes Alkenyl, optional substituiertes Alkenyloxy, optional substituiertes Aryl, optional substituiertes Aralkyl, optional substituiertes Aryloxy und optional substituiertes Aralkyloxy darstellen, und
die Reste Q Wasserstoff darstellen, und
alle in der obenstehenden Formel (I) umfassten nichtaromatischen cyclischen Reste optional einen oder mehrere Substituenten tragen können und/oder optional eine oder mehrere Doppelbindungen aufweisen können.

20. Epoxidharz nach Anspruch 19, wobei die Mischung aus polyphenolischen Verbindungen erhältlich ist durch ein Verfahren, umfassend das Kondensieren eines Dialdehyds eines Cycloalkans mit 6 Ringkohlenstoffatomen mit einer Phenol umfassenden phenolischen Verbindung.

21. Epoxidharz nach Anspruch 19 oder Anspruch 20, welches erhältlich ist durch ein Verfahren, umfassend das Inkontaktbringen der Mischung aus polyphenolischen Verbindungen mit Epichlorhydrin.

22. Epoxidharz nach einem der Ansprüche 19 bis 21, das erhältlich ist durch ein Verfahren, bei dem im Wesentlichen alle der phenolischen Hydroxygruppen in Glycidylethergruppen umgewandelt werden.

## Revendications

1. Mélange qui comprend :
(i) au moins un élément parmi (a) le mélange de composés polyphénoliques et/ou une forme prépolymérisée de ceux-ci et (b) une résine époxy et/ou une forme prépolymérisée de celle-ci et
(ii) au moins un élément parmi (c) une résine novolaque et (d) une résine époxy qui est différente de (b) ;
dans lequel le mélange de composés poly-phénoliques (a) peut être obtenu par un procédé comprenant la condensation d'un dialdéhyde de cycloalcane ayant d'environ 5 à environ 24 atomes de carbone dans le cycle avec un composé phénolique selon un rapport groupes hydroxyle phénoliques/groupes aldéhyde qui donne un mélange de composés polyphénoliques qui comprend au moins environ 20 % en poids de composé polyphénolique de formule (I) : 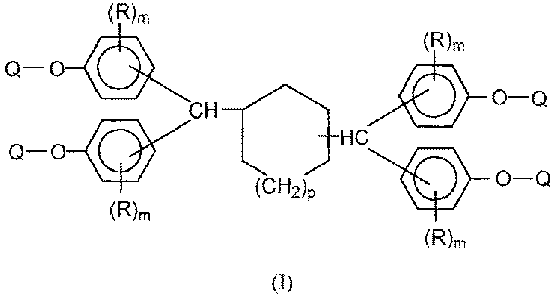 où :
p est égal à 0 ou un entier allant de 1 à environ 19 ; chaque m est indépendamment égal à 0, 1 ou 2 ;
les groupements R représentent indépendamment un halogène, les groupes cyano, nitro, hydroxyle, alkyle facultativement substitué, cycloalkyle facultativement substitué, alcoxy facultativement substitué, alcényle facultativement substitué, alcényloxy facultativement substitué, aryle facultativement substitué, aralkyle facultativement substitué, aryloxy facultativement substitué et aralkyloxy facultativement substitué ; et
les groupements Q représentent un hydrogène ;
et tout autre groupement cyclique non aromatique inclus dans la formule (I) ci-dessus peut être facultativement porteuse d'un ou de plusieurs substituants et/ou peut facultativement comprendre une ou plusieurs liaisons doubles ;
et dans lequel la résine époxy (b) est obtenue en transformant partiellement ou complètement les groupes hydroxyle phénoliques du mélange de composés polyphénoliques (a) en groupes glycidyl éther.

2. Mélange selon la revendication 1, dans lequel le mélange de composés polyphénoliques comprend au moins environ 50 % en poids du composé polyphénolique de formule (I).

3. Mélange selon l'une quelconque des revendications 1 et 2, dans lequel le rapport molaire composé phénolique/dialdéhyde de cycloalcane dans le procédé est d'environ 5/1.

4. Mélange selon l'une quelconque des revendications 1 à 3, dans lequel le cycloalcane a 6, 7 ou 8 atomes de carbone dans le cycle.

5. Mélange selon l'une quelconque des revendications 1 à 4, dans lequel le dialdéhyde dans le procédé comprend un dicarboxaldéhyde de cyclohexane.

6. Mélange selon l'une quelconque des revendications 1 à 5, dans lequel le composé phénolique dans le procédé comprend du phénol.

7. Mélange selon l'une quelconque des revendications 1 à 6, dans lequel la résine époxy (b) est obtenue en transformant substantiellement tous les groupes hydroxyle phénoliques en groupes glycidyl éther.

8. Mélange selon l'une quelconque des revendications 1 à 7, dans lequel la résine époxy (d) comprend une résine novolaque époxydée.

9. Mélange selon l'une quelconque des revendications 1 à 8, dans lequel le mélange comprend une résine époxy bromée.

10. Mélange selon l'une quelconque des revendications 1 à 9, le mélange comprenant en outre une ou plusieurs substances qui sont choisies parmi les catalyseurs de polymérisation, les agents de co-durcissement, les retardateurs de flamme, les synergistes pour retardateurs de flamme, les solvants, les charges, les fibres de verre, les promoteurs d'adhérence, les auxiliaires de mouillage, les auxiliaires de dispersion, les modificateurs de surface, les polymères thermoplastiques et les agents de démoulage.

11. Mélange selon l'une quelconque des revendications 1 à 10, le mélange étant partiellement ou complètement durci.

12. Produit qui comprend le mélange selon l'une quelconque des revendications 1 à 11.

13. Produit selon la revendication 12, dans lequel le produit est au moins choisi parmi un stratifié électrique, un substrat de CI, un moulage, un revêtement, une formulation pour fixage de puce et composé de moulage, un composite, une composition de remplissage et un adhésif.

14. Procédé pour préparer le mélange selon l'une quelconque des revendications 1 à 11.

15. Utilisation d'au moins un élément parmi (a) un mélange de composés polyphénoliques et/ou une forme prépolymérisée de ceux-ci et (b) une résine époxy et/ou une forme prépolymérisée de celle-ci pour améliorer au moins une caractéristique parmi la résistance thermique et la résistance de la résine ;
le mélange de composés polyphénoliques (a) pouvant être obtenu par un procédé comprenant la condensation d'un dialdéhyde de cycloalcane ayant d'environ 5 à environ 24 atomes de carbone dans le cycle avec un composé phénolique selon un rapport groupes hydroxyle phénoliques/groupes aldéhyde qui donne un mélange de composés polyphénoliques qui comprend au moins environ 20 % en poids du composé polyphénolique de formule (I) : 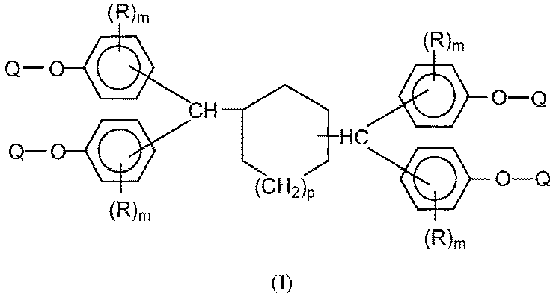 où :
p est égal à 0 ou un entier de 1 à environ 19 ; chaque m est indépendamment égal à 0, 1 ou 2 ;
les groupements R représentent indépendamment un halogène, les groupes cyano, nitro, hydroxyle, alkyle facultativement substitué, cycloalkyle facultativement substitué, alcoxy facultativement substitué, alcényle facultativement substitué, alcényloxy facultativement substitué, aryle facultativement substitué, aralkyle facultativement substitué, aryloxy facultativement substitué et aralkyloxy facultativement substitué ; et
les groupements Q représentent un hydrogène ;
et toute autre groupement cyclique non aromatique inclus dans la formule (I) ci-dessus peut être facultativement porteuse d'un ou de plusieurs substituants et/ou peut facultativement comprendre une ou plusieurs liaisons doubles ; et
la résine époxy (b) étant obtenue en transformant partiellement ou complètement les groupes hydroxyle phénoliques du mélange de composés polyphénoliques (a) en groupes glycidyl éther ; et
la résine étant fabriquée à partir d'au moins un composant parmi (c) une résine novolaque et (d) une résine époxy qui est différente de (b).

16. Utilisation selon la revendication 15, dans laquelle le matériau résultant du procédé comprend 5 % à 95 % en poids du mélange de composés polyphénoliques et/ou de la résine époxy de la présente invention, sur la base du poids total des composants de la résine.

17. Utilisation selon l'une quelconque des revendications 15 ou 16, dans laquelle p est égal à 1.

18. Utilisation selon l'une quelconque des revendications 15 à 17, dans laquelle la résine époxy (d) comprend une résine novolaque époxydée.

19. Résine époxy qui peut être obtenue par un procédé comprenant la transformation partielle ou totale des groupes hydroxyle phénoliques du mélange de composés polyphénoliques en groupes glycidyl éther ;
le mélange de composés polyphénoliques pouvant être obtenu par un procédé comprenant la condensation d'un dialdéhyde de cycloalcane ayant 6 atomes de carbone dans le cycle avec un composé phénolique selon un rapport groupes hydroxyle phénoliques/groupes aldéhyde qui donne un mélange de composés polyphénoliques qui comprend au moins environ 20 % en poids du composé polyphénolique de formule (I) : 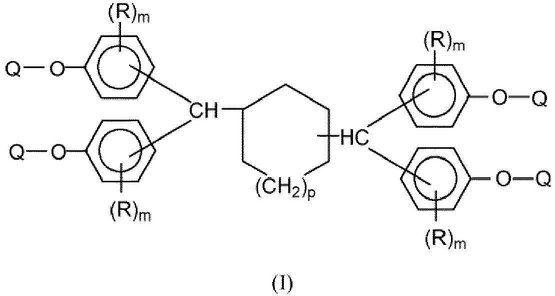 où :
p est égal à 1 ;
chaque m est indépendamment égal à 0, 1 ou 2 ;
les groupements R représentent indépendamment un halogène, les groupes cyano, nitro, hydroxyle, alkyle facultativement substitué, cycloalkyle facultativement substitué, alcoxy facultativement substitué, alcényle facultativement substitué, alcényloxy facultativement substitué, aryle facultativement substitué, aralkyle facultativement substitué, aryloxy facultativement substitué et aralkyloxy facultativement substitué ; et
les fractions Q représentent l'hydrogène ;
et tout autre groupement cyclique non aromatique inclus dans la formule (I) ci-dessus peut être facultativement porteuse d'un ou de plusieurs substituants et/ou peut facultativement comprendre une ou plusieurs liaisons doubles.

20. Résine époxy selon la revendication 19, dans laquelle le mélange de composés polyphénoliques peut être obtenu par un procédé comprenant la condensation d'un dialdéhyde de cycloalcane ayant 6 atomes de carbone dans le cycle avec un composé phénolique qui comprend du phénol.

21. Résine époxy selon la revendication 19 ou la revendication 20, qui peut être obtenue par un procédé comprenant la mise en contact du mélange de composés polyphénoliques avec de l'épichlorhydrine.

22. Résine époxy selon l'une quelconque des revendications 19 à 21, qui peut être obtenue par un procédé dans lequel substantiellement tous les groupes hydroxyle phénoliques sont transformés en groupes glycidyl éther.
